# EUROPÄISCHE PATENTSCHRIFT

(11) **EP 2 361 138 B1**
(45) Veröffentlichungstag und Bekanntmachung des Hinweises auf die Patenterteilung: **05.09.2012**
(21) Anmeldenummer: 09795903.5
(22) Anmeldetag: 28.11.2009
(51) Int. Cl.: B01D 53/14

(54) **VERFAHREN UND ANLAGE ZUR REINIGUNG VON ROHGASEN, INSBESONDERE BIOGAS, ZUR GEWINNUNG VON METHAN**
METHOD AND UNIT FOR PURIFYING RAW GASES, PARTICULARLY BIOGAS, FOR OBTAINING METHANE
PROCÉDÉ ET DISPOSITIF POUR ÉPURER DES GAZ BRUTS, EN PARTICULIER UN BIOGAZ POUR OBTENIR DU MÉTHANE

(30) Priorität: 03.12.2008 DE 102008060310
(43) Veröffentlichungstag der Anmeldung: 31.08.2011
(73) Patentinhaber: DGE Dr.-Ing. Günther Engineering GmbH, 06886 Wittenberg (DE)
(72) Erfinder: GÜNTHER, Lothar, 82538 Geretsried (DE)
(74) Vertreter: Tragsdorf, Bodo
(86) Internationale Anmeldenummer: PCT/EP2009/008487
(87) Internationale Veröffentlichungsnummer: WO 2010/063419

(56) Entgegenhaltungen:
- EP-A1- 1 634 946
- DE-A1- 10 356 276
- US-A1- 2006 213 370
- G. HOCHGESANG: "Anwendung von Absorptionsverfahren für die CO2-Entfernung ais Natur- und Synthesegasen" CHEMIE-INGENIEUR-TECHNIK, Bd. 40, Nr. 9/10, 1968, XP002576942

## Beschreibung

Die Erfindung bezieht sich auf ein Verfahren zur Reinigung von Rohgasen, insbesondere Biogas, zur Gewinnung von Methan, wobei im Roh- oder Biogas enthaltene Bestandteile, wie Kohlendioxid, Schwefelverbindungen, Ammoniak, in mehreren unterschiedlichen Verfahrensstufen abgetrennt werden, sowie eine zur Durchführung des Verfahrens geeignete Anlage.

Biogas entsteht durch anaerobe (sauerstofffreie) Vergärung von organischem Material und wird als regenerative Energiequelle genutzt. In Abhängigkeit von den jeweiligen Ausgangsstoffen, wie z.B. Klärschlamm, Gülle, Mist, Abfallstoffe pflanzlicher oder tierischer Herkunft, biologische Rohstoffe, wird in Klärgas, Faulgas, Deponiegas und Biogas unterschieden. Unter Biogas sollen nachfolgend alle vorgenannten Gase verstanden werden. Als Rohgase kommen solche in Frage, die als Hauptbestandteile CH₄ und CO₂ enthalten, wie z.B. Grubengas oder Flare-Gas (Erdölbegleitgas).

Biogase enthalten als Hauptbestandteile Methan und Kohlendioxid sowie weitere Nebenbestandteile, wie Stickstoff, Schwefelverbindungen, Sauerstoff, Wasserstoff und Ammoniak. Zur weiteren Nutzung von im Biogas enthaltenen Methan ist es daher erforderlich, das Biogas mehrstufig zu behandeln, um die unerwünschten Komponenten abzutrennen.

An sich übliche Verfahrensstufen, die in der Regel getrennt erfolgen, sind die Entfeuchtung (Abtrennung von Wasser), Entschwefelung, Abtrennung von Kohlendioxid und Ammoniak.

Zur Entschwefelung sind biologische Verfahren (unter Verwendung von Mikroorganismen) sowie chemische Adsorptionsverfahren bekannt, wobei der Schwefelwasserstoff auf unterschiedlichen Wegen zu elementarem Schwefel umgesetzt wird.

Die Abtrennung von Kohlendioxid sowie geringer Mengen an Schwefelwasserstoff erfolgt z.B. mittels Druckwasserwäsche, Membranverfahren, Selexol-Verfahren (unter Hochdruck), Druckwechseladsorption oder Aminwäsche (auf physikalischem oder chemischem Weg). Einige dieser Verfahren ermöglichen noch die zusätzliche Abtrennung von Wasser bzw. Ammoniak.

Die meisten der vorgenannten Verfahren sind energieaufwendig und führen zu Methanverlusten.

Bei der Druckwasserwäsche und Druckwechseladsorption treten relativ hohe Methanverluste auf, die ca. 2 bis 5% des im Biogas enthaltenen Methans betragen. Hinzu kommt, dass dieses Methan im abgeschiedenen Kohlendioxid vorliegt und somit bei den kleinen Konzentrationen nur mittels einer Zusatzfeuerung als Brennstoff verwertbar ist. Bei der Druckwechseladsorption fällt weiter systembedingt die Methanemission mit starken Schwankungen an und erfordert eine Glättung. Außerdem darf das Rohgas nur eine sehr geringe H₂S-Konzentration aufweisen und erfordert eine aufwendige Entsorgung der eingesetzten Aktivkohle.

Eine Wäsche mit einer Waschlösung, wie z.B. einer Aminwäsche, ist nur dann wirtschaftlich vertretbar, wenn die verunreinigte Waschlösung wieder regeneriert wird.

Aus der DE 10 2005 051 952 B3 ist ein Verfahren zur Herstellung von Methan und flüssigem Kohlendioxid aus Raffinerie- und/oder Biogas bekannt. Das Rohgas wird in einer Vorstufe gereinigt (Entfernung von Verunreinigungen wie NH₃, H₂SO₄, H₂S, SO₂ und COS) und anschließend einer Absorptionskolonne zugeführt, in der bei einem Druck von vorzugsweise 5 bis 30 bar unter Verwendung einer aminhaltigen Waschlösung im Rohgas enthaltenes Kohlendioxid in der Waschlösung gebunden wird. Das anfallende gereinigte Gas enthält ca. 98 Vol.-% Methan und kann einer direkten Weiterverwendung zugeführt werden. Die verunreinigte Waschlösung wird unter Druck und bei erhöhten Temperaturen (180 bis 230 °C) in einer Desorptionskolonne regenerativ aufbereitet.

Die Verfahrensweise unter Druck erfordert einen hohen apparativen Aufwand.

Aus der WO 2008/034473 A1 ist ein Verfahren zur Trennung von Methan und Kohlendioxid aus Biogas bekannt, das eine drucklose Entfernung von Kohlendioxid ermöglicht und bei dem Methangas mit einer Reinheit von über 99,5 % anfällt.

Wie bei allen Aminwäschen wird zur Regenerierung der Waschlösung eine relativ hohe Energie verbraucht, die im Bereich von 0,5 bis 0,8 kWh/Nm³ Biogas liegt.

In der DE 103 56 276 A1 ist ein Verfahren zur Reinigung von Biogas zur Gewinnung von Methan offenbart. Gemäß diesem Verfahren werden im Biogas vorhandene wasserlösliche Bestandteile in einem mehrstufigen Reinigungsprozess abgetrennt, wobei Wasser als Lösungsmittel eingesetzt werden kann.

Der Reinigungsprozess umfasst dann mindestens drei unmittelbar nacheinander folgenden Reinigungsstufen, wobei im Kreislauf geführtes zusatzfreies Frischwasser verwendet wird.

In der ersten Reinigungsstufe durchströmt das zu reinigende Biogas bei Überdruck von z.B. 6 bar eine Waschkolonne mit Füllkörperschüttung im Gegenstrom zum zugeführten Frischwasser, wobei im Frischwasser Kohlendioxid, Schwefelwasserstoff gebunden werden und am Kopf der Waschkolonne Methangas mit einem Methangehalt von z.B. 97% abgezogen wird. In der zweiten Reinigungsstufe wird aus dem aus der Waschstufe abgeführten verunreinigten Waschwasser in einer Entspannungssäule bei Temperaturen bis 80°C im Waschwasser gelöstes Methan nahezu vollständig abgetrennt. In der dritten Reinigungsstufe wird aus dem aus der zweiten Reinigungsstufe abgeführten verunreinigten Waschwasser in einer Strippkolonne mit Füllkörperschüttung oder Packung im Gegenstrom zur Strippluft Kohlendioxid abgetrennt und als Abgas abgelassen. Das gereinigte Waschwasser wird der Waschstufe wieder zugeführt.

Der Erfindung liegt die Aufgabe zugrunde, ein Verfahren zur Reinigung von Roh- oder Biogas zur Gewinnung von Methan, zu schaffen, das sich durch einen geringen Energieverbrauch auszeichnet und eine Erhöhung des Methangehaltes um mindestens 10 % ermöglicht, bei geringen Methanverlusten. Ferner soll eine zur Durchführung des Verfahrens geeignete Anlage geschaffen werden.

Erfindungsgemäß wird die Aufgabe durch die im Anspruch 1 angegebenen Merkmale gelöst. Vorteilhafte Ausgestaltungen der Verfahrensweise sind Gegenstand der Ansprüche 2 bis 10. Die Merkmale einer zur Durchführung des Verfahrens geeigneten Anlage sind in Anspruch 11 angegeben. Vorteilhafte Weiterbildungen dieser Anlage sind Gegenstand der Ansprüche 12 bis 15.

Gemäß der vorgeschlagenen Verfahrensweise erfolgt der Reinigungsprozess in mindestens drei unmittelbar nacheinander folgenden Reinigungsstufen und unter Verwendung von im Kreislauf geführten zusatzfreien Frischwasser. Als Frischwasser kann aus dem örtlichen Versorgungsnetz stammendes Wasser oder Brunnenwasser oder aufbereitetes Regenwasser eingesetzt werden. Das verwendete Wasser enthält keine Zusätze. Die unbedingt erforderlichen drei Reinigungsstufen sind folgende:
Das aus einer Biogasanlage oder anderen Anlage, z.B. für Faulgas, Klärgas oder Deponiegas, abgeführte zu reinigende Roh- oder Biogas durchströmt unter Normaldruck oder bei einem Überdruck von bis zu 6 bar eine Waschkolonne mit Füllkörperschüttung im Gegenstrom zum zugeführten Frischwasser. Dabei werden im Frischwasser Kohlendioxid, Schwefelwasserstoff, Ammoniak, und andere im Gas enthaltene organische wasserlösliche Stoffe gebunden. Vorzugsweise findet in der ersten Reinigungsstufe eine Entschwefelung des Roh- oder Biogases statt. Am Kopf der Waschkolonne wird Methangas mit einem Methangehalt von mindestens 65 % abgezogen.

Diese Gaswäsche wird im Regelfall unter Normaldruck durchgeführt. In Ausnahmefällen kann jedoch auch mit Überdruck bis zu 3 oder 4 höchsten jedoch 6 bar gearbeitet werden. Bei einem höheren Druck löst sich eine größere Menge an Kohlendioxid im Waschwasser, bei 3 bar eben 3-mal so viel. Damit ist eine um den Faktor 3 kleinere Waschwassermenge erforderlich und die Waschkolonne kann durch das geringere Gasvolumen kleiner gebaut werden. Alle konventionellen Druckgaswäsche-Verfahren benötigen einen Druck von über 6 bar, um wirtschaftlich Methankonzentrationen von über 96 Vol.-% zu erhalten. Ein höherer Druck führt jedoch zu einem deutlich höheren Energieverbrauch, da nachträglich wieder entspannt werden muss. Außerdem entstehen höhere Methanverluste. Wesentlich für eine erfolgreiche Verfahrensdurchführung sind die nachfolgenden zwei Reinigungsstufen mittels Strippkolonnen.

In einer ersten Strippkolonne mit Füllkörperschüttung oder Packung wird das aus der Waschstufe abgeführte verunreinigte Waschwasser unter Zuführung von Strippluft; Strippluft und Sauerstoff; oder Strippluft und Kohlendioxid; in einer Menge von 0,1 bis 20 %, bezogen auf die Menge an zugeführtem Gas, unter Normaldruck, im Gegenstromprinzip, bei Temperaturen von bis zu 80 °C, gereinigt, wobei im Waschwasser gelöstes Methan nahezu vollständig (mindestens 90 %) aus diesem abgetrennt wird. Dabei entsteht als Abgas ein sauerstoffhaltiges Strippgas in Brenngasqualität, bei Zuführung von Luft oder Sauerstoff zum Waschwasser.

Durch den Zusatz von Kohlendioxid, das vorzugsweise aus dem Rohgas stammt, wird kein Fremdgas zugeführt.

Das in der zweiten Reinigungsstufe entstehende Abgas kann entweder in den Fermenter der Biogasanlage zurückgeführt oder dem aus der Waschstufe abgetrennten Methangasstrom zur Anreicherung des Methangehaltes zugeführt oder als Brenngas genutzt werden.

Vorzugsweise kann die erste Strippkolonne auch zweistufig ausgeführt werden, wobei in der ersten Stufe Sauerstoff und in der zweiten Stufe Strippluft, oder in umgekehrter Reihenfolge, zugeführt werden, auch in Kombination mit Kohlendioxid. Dadurch werden zwei unterschiedliche Brenngase mit unterschiedlichen Sauerstoffgehalten erhalten. Das sauerstoffreiche Brenngas kann z.B. als Sauerstoffquelle für eine biologische Entschwefelung des Biogases, entweder im Fermenter oder außerhalb, genutzt werden.

Alternativ kann bei der Kombination mit Kohlendioxid das erhaltene Brenngas im Kreislauf zum Fermenter gefahren werden, ohne dass Luft oder Sauerstoff eingetragen wird.

Über den Zusatz von CO₂ kann auch mit CO₂ angereichertes Brenngas erhalten werden. In der dritten Reinigungsstufe wird das aus der ersten Strippkolonne abgeführte verunreinigte Waschwasser in einer zweiten Strippkolonne mit Füllkörperschüttung oder Packung unter Vakuum, vorzugsweise im Gegenstromprinzip oder gegebenenfalls auch im Gleichstromprinzip, gereinigt, wobei im Waschwasser gelöstes Kohlendioxid mindestens bis auf einen Restgehalt von unter 300 mg/l abgetrennt wird.

Der Druck für das Vakuum liegt vorzugsweise im Bereich bis 0,01 bar. Je höher das Vakuum, umso geringer ist der Restgehalt an gelöstem CO₂ im Waschwasser. Bei einem Vakuum von ca. 0,5 bar wird der angestrebte Restgehalt an CO₂ erreicht. Das angelegte Vakuum sollte mindestens 100 mbar betragen.

Die Behandlung des verunreinigten Waschwassers erfolgt in dieser Stufe ohne Zusatz von Strippluft oder nur geringen Mengen an Strippluft.

Durch eine Fahrweise unter Vakuum wird verhindert, dass sich Strippgas im Waschwaser löst bzw. das Löslichkeitsvermögen wird deutlich reduziert. Dies betrifft CO₂, Luft, Sauerstoff und Methan. Mit dem Anlegen von Vakuum kann der CO₂-Gehalt im Waschwasser ohne Luftzusatz gesenkt werden. Im Waschwasser können nur noch sehr geringe Mengen an Luft gelöst werden,

Im Gegensatz dazu lässt sich bei einer Fahrweise unter Normaldruck das im Waschwasser gelöste CO₂ nur schwer abtrennen. Außerdem ist bei Normaldruck die Verwendung von Luft als Strippmedium erforderlich. Dabei besteht die Gefahr, dass sich Luft im Wasser löst und gelöste Luft in der Waschkolonne in das Biomethan ausgestrippt wird.

Das in der dritten Stufe im Vakuumstripper gereinigte Waschwasser wird wieder in die Waschstufe des Gaswäschers zurückgeführt und das Abgas wird an die Umgebung abgelassen oder anderweitig verwertet.

Die vorgeschlagene Verfahrensweise führt zu vergleichsweise geringen Methanverlusten, die unter 0,05 % liegen. Bei einer Fahrweise bei Normaldruck liegt der Energieverbrauch für die drei Reinigungsstufen unter 0,04 kWh/Nm³ Biogas. Dadurch wird eine besonders hohe Wirtschaftlichkeit erzielt. Hinzu kommt noch die Möglichkeit der energetischen Nutzung des in der ersten Strippstufe anfallenden Abgases, das in Brenngasqualität vorliegt. Dies ist insbesondere dann von Bedeutung, wenn Biogas zur Einspeisung in ein Erdgasnetz oder zur Erzeugung von Kraftstoff verwendet werden soll. In diesem Fall steht keine Abwärme aus einer Verstromung zur Verfügung. Die Abwärme aus einer Biomethanverdichtung ist für die Fermenterheizung nicht ausreichend. In diesem Fall muss zusätzlicher fossiler Brennstoff bereitgestellt werden. Das als Nebenprodukt erzeugte Brenngas kann zweckmäßigerweise für die Fermenterbeheizung genutzt werden.

Alternativ kann das aus der Waschkolonne abgezogene gereinigte Biogas zur Erhöhung der Methankonzentration und der Speicherkapazität des Biogases im Fermenter direkt in den Fermenter der Biogasanlage eingeleitet werden.

Durch diese Kopplung des erfindungsgemäßen Verfahrens mit einer Biogasanlage kann im Fermenter ein Biogas mit deutlich höherem Methangehalt erzeugt und die Speicherkapazität für das Biogas enorm erweitert werden. Das aus dem Fermenter abgezogene Biogas mit einer erhöhten Methankonzentration kann dann ohne weitere Aufarbeitung direkt einer wirtschaftlichen Verwertung zugeführt werden.

Das aus der Waschstufe abgezogene gereinigte Biogas (Methangas) besitzt bereits eine ausreichende Reinheit für eine direkte weitere Verwendung, z.B. für eine Einspeisung in Erdgasnetze oder zum Betreiben von Blockheizkraftwerken. Werden höhere Erdgasqualitäten verlangt, so kann das vorliegende Methangas durch eine weitere Aufbereitung bzw. Reinigung mittels einer Aminwäsche an die geforderten Qualitäten angepasst werden. Das Methangas kann entweder einzeln oder zusammen mit dem aus der ersten Strippkolonne abgezogenen Strippgas (Brenngas) einer weiteren Aufbereitung zur Erhöhung des Methangehaltes zugeführt werden. Da der größte Teil der Verunreinigungen bereits aus dem Biogas entfernt wurde, können eine nachfolgende Aminwäsche sowie die Regenerierung der Waschlösung mit deutlich geringerem Aufwand an Energie und bei deutlich geringeren Methanverlusten vorgenommen werden.

Das Frischwasser wird der ersten Reinigungsstufe, der Waschkolonne mit einer Temperatur von bis zu 65 °C, vorzugsweise unter 20 °C, zugeführt. Als Frischwasser kann Grundwasser mit einer Temperatur von 10 bis 15 °C verwendet werden.

Je niedriger die Temperatur des Waschwassers, desto höher ist die Trennleistung für Kohlendioxid. Bei warmen Außentemperaturen sollte daher das Waschwasser vor Einleitung in den Gaswäscher gekühlt werden. Über die Parameter Waschwassermenge/h und Waschwassertemperatur in der Waschkolonne kann die Trennleistung für im Waschwasser gelöstes Kohlendioxid eingestellt werden. Eine höhere Waschwassermenge und eine tiefere Waschwassertemperatur führen zu einer höheren Trennleistung.

In der ersten Strippstufe sollte das Verhältnis Strippluftmenge:Biogasmenge (Rohgas) 1:50 bis 1:1000, vorzugsweise 1:100, betragen. Bei einem kleinen Verhältnis von 1:50 wird im Strippgas (Abgas) eine größere Methankonzentration erzielt als bei größeren Verhältnissen. Dabei ist zu berücksichtigen, dass gegebenenfalls der Methanschlupf ansteigt. Als Strippluft wird vorzugsweise normale Luft eingesetzt, wobei jedoch auch Sauerstoff und Stickstoff oder Kohlendioxid geeignet sind, entweder einzeln oder als Gemisch.

Das zugeführte Biogas sollte vor der Einleitung in die Waschstufe bzw. den Gaswäscher auf einen Schwefelgehalt von < 5 ppm eingestellt werden. Dies kann durch eine an sich bekannte Entschwefelung im Fermenter oder mittels einer separaten Vorentschwefelung erfolgen. Bei einem zu hohen Schwefelgehalt z.B. von über 30 ppm im verunreinigten Waschwasser der Waschstufe kann es erforderlich sein, das im Kreislauf gefahrene Waschwasser teilweise oder vollständig durch Frischwasser zu ersetzen. Um dies zu umgehen, kann eine Teilmenge des am Sumpf der zweiten Strippkolonne abgezogenen Waschwassers ausgekreist, dieser ein Schwefelwasserstoff bindendes Reaktionsmittel, wie z.B. Eisen-III-Chlorid oder Eisen-III-Oxid, zugesetzt werden, wodurch gelöster Schwefelwasserstoff chemisch gebunden, und nach dem Ausfällen des Eisen(II)-disulfids das Waschwasser wieder in den Kreislauf zurückgeführt wird. Bei höheren Konzentrationen an Schwefelwasserstoff im Biogas als 30 ppm kann die Gaswäsche gleichzeitig zur externen Entschwefelung genutzt werden. Dabei ist dem Strippgas aus der zweiten Strippstufe eine entsprechende Entschwefelung, wie z.B. über Biofilter, nachzuschalten. Die zur Durchführung des Verfahrens vorgeschlagene Anlage zeichnet sich durch einen einfachen und kostengünstigen Aufbau aus und wird nachfolgend näher erläutert.

In der zugehörigen Zeichnung zeigen:
- Fig. 1: eine erste Ausführungsvariante einer Anlage zur Durchführung des Verfahrens in vereinfachter Darstellung und
- Fig. 2: eine zweite Ausführungsvariante der Reinigungseinheit A in vereinfachter Darstellung.

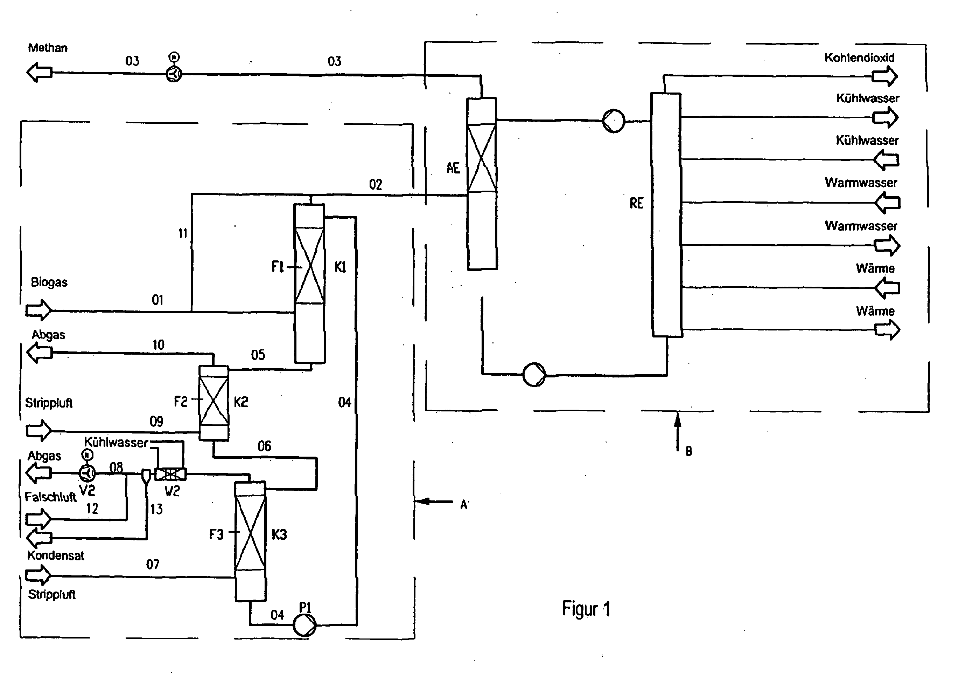

Die in Figur 1 gezeigte Anlage besteht aus einer erfindungsgemäßen Reinigungseinheit A zur Gewinnung von Methan aus Roh- oder Biogas und einer wahlweise zuschaltbaren Baugruppe B für eine nachträgliche Aminwäsche in an sich bekannter Art. Die Baugruppe B für die Aminwäsche umfasst als Hauptbestandteile eine Absorptionseinheit AE zur weiteren Abtrennung von Kohlendioxid aus dem in der Reinigungseinheit A vorgereinigten Gas und eine Regenerationseinheit RE für die Regenerierung der anfallenden verunreinigten aminhaltigen Waschlösung, die im Kreislauf gefahren wird.

Die Reinigungseinheit A besteht aus drei in Reihe geschalteten Reinigungskolonnen, einer Waschkolonne (Gaswäscher) K1, einer ersten Strippkolonne K2 und einer zweiten Strippkolonne K3 als Vakuumkolonne, wobei in der Waschkolonne K1 im Biogas (Rohgas) enthaltene Bestandteile, wie Kohlendioxid, Schwefelverbindungen, Ammoniak und andere wasserlösliche Stoffe, abgetrennt werden. Die Waschkolonne K1 besteht aus einem Waschturm mit einer Füllkörperschüttung oder Packung F1 aus Polyethylenpartikeln mit einer Oberfläche von 200 bis 850 m²/m³ und einer Schütthöhe von 2 bis 16 m, je nach gewünschten Grad an CO₂-Abtrennung.

Die erste Strippkolonne K2 und die zweite Strippkolonne K3 bestehen jeweils aus einem Turm mit einer Füllkörperschüttung F2 bzw. F3 aus Polyethylenpartikeln. Die erste Strippkolonne K2 enthält Polyethylenpartikel mit einer Oberfläche von 250 bis 900 m²/m³, vorzugsweise 300 bis 790 m²/m³, und einer Schütthöhe von 2 bis 4 m. In der zweiten Strippkolonne K3 beträgt die Schütthöhe 2 bis 8 m, wobei Füllkörper aus Edelstahl- oder Polyethylenpartikeln mit einer Oberfläche von 100 bis 480 m²/m³ zum Einsatz kommen. Die zweite Strippkolonne K3 ist als Vakuumstrippkolonne ausgelegt und besteht vorzugsweise aus Edelstahl.

Die Reinigungskolonnen K1, K2 und K3 stehen über eine Kreislaufleitung 05, 06, 04 miteinander in Verbindung, wobei in die Leitung 04 eine Kreislaufpumpe P1 eingebunden ist, mittels der zugeführtes Waschwasser, das aus einem Brunnen oder dem örtlichen Versorgungsnetz oder einer Regenwasseraufbereitung stammt, im Kreislauf gefahren wird. Das zu reinigende Biogas wird über die Leitung 01 unterhalb der Füllkörperschüttung F1 in die Waschkolonne K1 eingeleitet. Waschwasser wird am Kopf der Waschkolonne K1 über die Leitung 04 zugeführt und durchströmt im Gegenstrom zu dem zugeführten Biogas die Füllkörperschüttung bzw. Packung F1. Am Kopf der Waschkolonne K1 wird über die Leitung 02 gereinigtes Biogas (Methangas) abgezogen. Am Sumpf der Waschkolonne K1 wird über die Leitung 05 verunreinigtes Waschwasser abgeführt und am Kopf der ersten Strippkolonne K2 in diese eingeleitet. Unterhalb der Füllkörperschüttung F2 der Strippkolonne K2 gelangt in diese über die Leitung 09 ein erster Strippluftstrom. Das entstehende Strippgas (Abgas) wird am Kopf der Strippkolonne K2 über die Leitung 10 abgezogen. Am Sumpf der Strippkolonne K2 anfallende verunreinigte Waschlösung wird über die Leitung 06 abgezogen und am Kopf der zweiten Strippkolonne K3 in diese eingeleitet.

Der Druck der in der Leitung 06 abgeführten Waschlösung ist dem in der Vakuumstrippkolonne K3 herrschenden Unterdruck angepasst abgetaucht, so dass in dieser kein Strippgas aus der Strippkolonne 2 durchschlagen kann (0,1 bar = 1 m Abtauchung).

Unterhalb der Füllkörperschüttung F3 der Vakuumstrippkolonne K3 wird über die Leitung 07 ein zweiter Strippgasstrom (z.B. Luft und/oder Kohlendioxid) zugeführt. Das anfallende Strippgas (Abgas) wird am Kopf der Vakuumstrippkolonne K3 über die Leitung 08 und die in diese eingebundene Vakuumpumpe V2 abgeführt.

Über die Vakuumpumpe V2 wird das in der zweiten Strippkolonne K3 benötigte Vakuum erzeugt. Zur Einstellung des jeweils gewünschten Vakuums in der Vakuumkolonne K3 wird der Vakuumpumpe V2 über die Leitung 12 Falschluft zugemischt. Diese Falschluft kann zur Vermeidung einer Kondensation vorgewärmt werden.

Außerdem ist in die Leitung 08 noch ein Wärmetauscher W2 eingebunden, um das am Kopf der Vakuumkolonne K3 abgezogene Strippgas zu kühlen und zu entfeuchten, wobei das entstehende Kondensat über die Leitung 13 abgeführt wird.

Das am Sumpf der Strippkolonne K3 anfallende gereinigte Waschwasser wird über die Leitung 04 zum Kopf der ersten Waschkolonne K1 gepumpt. Der Kontakt zwischen Strippgas und Waschwasser in den Strippkolonnen K2 und K3 erfolgt im Gegenstrom oder gegebenenfalls auch im Gleichstrom (Strippkolonne K3).

In die Leitung 01 zur Zuführung von Biogas kann eine Baypassleitung 11 eingebunden werden, die mir der Absorptionseinheit AE verbunden ist.

Die Strippvorgänge werden unter Normaldruck (Strippkolonne K3) und Vakuum (Strippkolonne K3) durchgeführt.

Falls seitens des Betreibers der Wunsch nach weiterer Methananreicherung des über die Leitung 02 abgezogenen Methangases besteht, so kann dieses der nachgeschalteten Aminwäsche zugeführt werden (Baugruppe B). Das hochreine Methangas wird nach der Aminwäsche über die Leitung 03, in die ein Biomethanverdichter eingebunden ist, am Kopf der Absorptionseinheit AE abgezogen. Die Reinigungseinheit A kann auch ohne eine nachträgliche Aminwäsche betrieben werden.

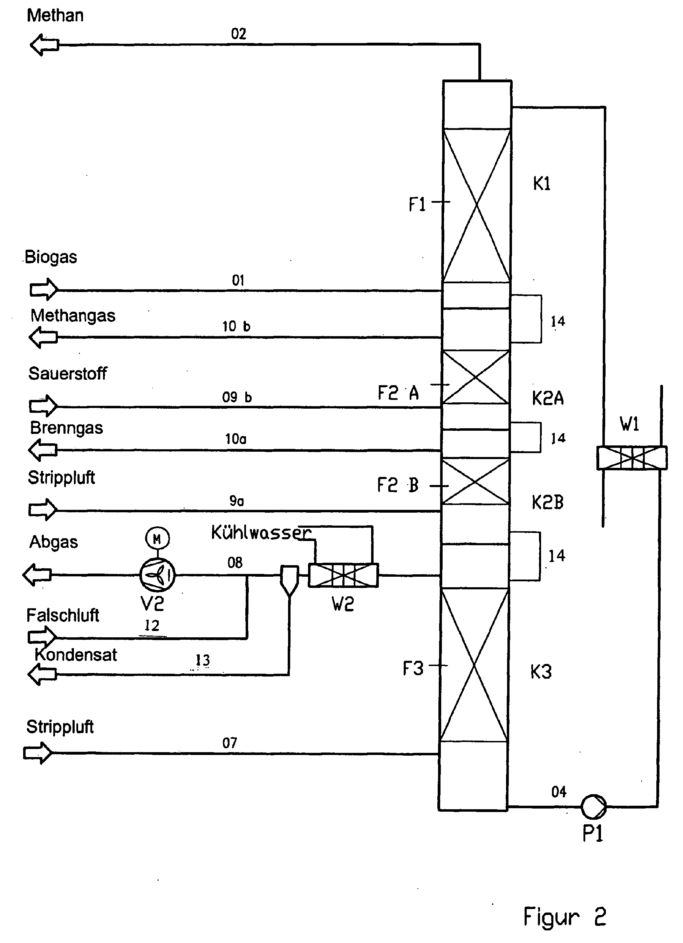

Die in Fig. 2 gezeigte Reinigungseinheit A unterscheidet sich nur dadurch von der Reinigungseinheit gemäß Fig. 1, dass die einzelnen Reinigungsstufen K1 bis K3 in einem einheitlichen Turm angeordnet sind und die Strippkolonne K2 zweiteilig ausgeführt ist, unterteilt in den oberen Kolonnenabschnitt K2A und den unteren Kolonnenabschnitt K2B, die je eine Füllkörperschüttung F2A bzw. F2B besitzen.

Dem Kolonnenabschnitt K2A werden über die Leitung 09b Sauerstoff und dem Kolonnenabschnitt K2B über die Leitung 09a Luft als Strippmedien zugeführt.

Z.B. werden dem Kolonnenabschnitt K2A nur 0,5 Nm³/h Sauerstoff zugeführt. Unter diesen Bedingungen werden 4 Nm³/h an gelöstem Methan aus dem Waschwasser entfernt. Über die Leitung 10b wird ein sauerstoffreiches Methangas abgezogen, das als Sauerstoffquelle für eine biologische Entschwefelung des Biogases (Rohgases) genutzt wird.

In dem nachgeschalteten Kolonnenabschnitt K2B wird aus dem verunreinigten Waschwasser das in diesem noch enthaltene restliche Methan mittels Luft entfernt. Das über die Leitung 10a abgeführte Brenngas wird einer thermischen Verwertung zugeführt.

Das anfallende verunreinigte Waschwasser wird durch je vier Überläufe 14 von der Waschkolonne K1 in die erste Strippkolonne K2 und von dieser in die zweite Strippkolonne K3 geleitet, die als Vakuumkolonne ausgeführt ist.

Die zwischen den einzelnen Kolonnen angeordneten Trennböden sind hinsichtlich Gasbeladung technisch dicht und hinsichtlich Flüssigkeitsbeladung voll durchlässig ausgeführt. Zusätzlich ist in die Kreislaufleitung 04 hinter der Pumpe P1 noch ein Wärmetauscher W1 zur Kühlung des Waschwassers eingebunden.

Die Betriebsweise der Anlage wird nachfolgend an einem Beispiel erläutert:
Aus dem Fermenter einer Biogasanlage stammendes Biogas, das bereits im Fermenter ohne Zuführung von Luft oder Sauerstoff vorentschwefelt wurde, hat folgende Zusammensetzung:

| | | |
|---|---|---|
| Methan | 52 | Vol.-% |
| Kohlendioxid | 44 | Vol.-% |
| Wasser | 3,4 | Vol.-% |
| Wasserstoff | 0,1 | Vol.% |
| Sauerstoff | 0,1 | Vol.% |
| Stickstoff | 0,4 | Vol.-% |
| H₂S | 3 | ppm |
| NH₃ | 20 | ppm |

Biogas (500 Nm³/h) mit einer Temperatur von 38 bis 45 °C wird direkt vom Fermenter der Waschkolonne K1 zugeführt, durchströmt die Füllkörperschüttung (Höhe 4 m) und gelangt dabei mit im Gegenstrom zugeführten, im Kreislauf geführten Waschwasser, das aus dem örtlichen Versorgungsnetz stammt, in Kontakt. Der Waschprozess erfolgt unter Normaldruck (-10 bis + 20 mbar), wobei, bezogen auf die zugeführte Menge an Biogas, 400 m³/h Wasser zugeführt werden. Das in der Waschkolonne K1 gereinigte Waschwasser hat eine Beladung von 300 mg/l an CO₂. Während der drucklosen Gaswäsche werden aus dem Biogas CO₂, H₂S und NH₃ entfernt und in dem Waschwasser gelöst. Der Anteil an gelöstem CO₂ im beladenen Waschwasser beträgt 800 mg/l. Damit werden 200 kg/h CO₂ im Waschwasser gelöst. Womit der entfernte Anteil an CO₂ ca. 46,4 % beträgt.

Am Kopf der Waschkolonne K1 wird gereinigtes Biogas (Methangas) in einer Menge von 333 Nm³/h mit folgender Zusammensetzung abgezogen:

| | | |
|---|---|---|
| Methan | 65,43 | Vol.-% |
| Kohlendioxid | 30,44 | Vol.-% |
| Wasser | 3,36 | Vol.-% |
| Wasserstoff | 0,13 | Vol.-% |
| Sauerstoff | 0,13 | Vol.-% |
| Stickstoff | 0,51 | Vol.-% |
| H₂S | <1 | ppm |
| NH₃ | <1 | ppm |

Das am Sumpf der Waschkolonne K1 anfallende verunreinigte Waschwasser, das mitgerissenes, im Waschwasser gelöstes Methan enthält (sogenannter Methanschlupf) wird direkt in einer nachfolgenden zweiten Reinigungsstufe, durch eine erste Strippkolonne K2 geleitet, in der mittels zugeführter Strippluft aus dem verunreinigten Waschwasser eine partielle Entfernung von Methan im Gegenstrom erfolgt. Der Strippvorgang in dieser zweiten Reinigungsstufe erfolgt unter Normaldruck.

Die zugeführte, geringe Menge Strippluft (5 Nm³/h) gewährleistet, dass unter den Bedingungen der Auslegung der ersten Strippkolonne (Füllkörperoberfläche 790 m²/m³; Schütthöhle 2 m) ca. 6,8 Nm³/h gelöstes Methan zu über 98 % aus dem verunreinigten Waschwasser mittels der Strippluft entfernt werden. Das am Kopf der ersten Strippkolonne K2 abgezogene Strippgas (Abgas) ist noch mit CO₂ (ca. 4 Nm³/h) beladen. Das anfallende Strippgas (Abgas) besitzt einen Methangehalt von 43 Vol.-% und entspricht in seiner Qualität einem vollwertigen Brenngas mit einem Heizwert von 74,5 kW.

Dieses kann zur Anreicherung des aus der Waschkolonne K1 abgeführten Methangasstromes eingesetzt oder energetisch als Brenn- bzw. Heizgas genutzt werden. Mittels der zweiten Reinigungsstufe wird somit sichergestellt, dass die Methanverluste an im Biogas enthaltenem Methan insgesamt relativ gering bleiben und einen Wert von 0,5 % nicht übersteigen.

Das in der ersten Strippstufe K2 anfallende verunreinigte, methanfreie Waschwasser wird unmittelbar einer weiteren Reinigungsstufe, der Vakuumstrippstufe K3 abgetaucht zugeführt, in der aufgrund des angelegten Vakuums (0,5 bar) im Waschwasser gelöstes CO₂ aus diesem entfernt wird.

Die Vakuumstrippkolonne K3 (Schüttkörperoberfläche 480 m²/m³; Schütthöhe 2 m) steht über die Leitung 08 mit einer Vakuumpumpe V2 in Verbindung. Über die Leitung 12 wird kontinuierlich Falschluft angesaugt und somit das Vakuum eingestellt.

Es werden 300 Nm³/h warme Strippluft (25 °C) zugeführt, die das im Waschwasser gebundene Kohlendioxid aufnimmt. Aufgrund des angelegten Vakuums wird verhindert, dass sich Strippgas im Waschwasser löst.

Die Kohlendioxidbeladung im Waschwasser wird unter diesen Bedingungen von etwa 780 g/l auf 300 mg/l reduziert. Das am Sumpf der Strippkolonne K3 anfallende, gereinigte Waschwasser wird mittels der Pumpe P1 über die Leitung 04 der Waschkolonne K1 zugeführt.

Das aus der Strippkolonne K3 austretende Abgas kann direkt und ohne Nachbehandlung in die Umgebung abgeleitet werden.

Für die gesamte Prozessführung der drei Reinigungsstufen K1, K2 und K3 sind nur 18,5 kW an Elektroenergie erforderlich, was für die Wirtschaftlichkeit des Verfahrens von wesentlicher Bedeutung ist. Dieser geringe Energieverbrauch bedeutet, bezogen auf die Einsatzmenge an Biogas (500 Nm³/h), einen spezifischen Verbrauch von 0,037kWh/Nm³. Das am Kopf der Waschkolonne K1 abgezogene gereinigte Biogas (Methangehalt 65,43 Vol.-%) kann direkt einer weiteren wirtschaftlichen Verwertung zugeführt werden oder bei Bedarf zur Erhöhung des Methangehaltes weiter aufgereinigt werden.

Die weitere Aufreinigung kann z.B. mittels einer an sich bekannten Aminwäsche erfolgen, wie z.B. in den Druckschriften DE 10 200 051 952 B3 und WO 2008/034473 A1 beschrieben.

Nach Aufreinigung des am Kopf der Waschkolonne K1 abgezogenen Methangases mittels einer Aminwäsche mit einem aminhaltigen Waschmittel wird ein gereinigtes Biogas (Methangas) mit folgender Zusammensetzung erhalten:

| | | |
|---|---|---|
| Methan | 88,3 | Vol.-% |
| Kohlendioxid | 0,3 | Vol.-% |
| Wasser | 10,3 | Vol.-% |
| Wasserstoff | 0,17 | Vol.-% |
| Sauerstoff | 0,17 | Vol.-% |
| Stickstoff | 0,69 | Vol.-% |
| H₂S | 2 | ppm |
| NH₃ | 1 | ppm |

Durch eine nachgeschaltete Entfeuchtungsstufe wird noch im Biogas enthaltenes Wasser abgetrennt und das gereinigte Biogas auf einen Taupunkt von 2 °C eingestellt. Danach hat das Biogas folgende Zusammensetzung:

| | | |
|---|---|---|
| Methan | 97,7 | Vol.-% |
| Kohlendioxid | 0,38 | Vol.-% |
| Wasser | 0,78 | Vol.-% |
| Wasserstoff | 0,19 | Vol.-% |
| Sauerstoff | 0,19 | Vol.-% |
| Stickstoff | 0,76 | Vol.-% |
| H₂S | 2 | ppm |
| NH₃ | 1 | ppm |

Durch weitere Kühlung und Entfernung des restlichen Wasseranteiles und/oder die Reduzierung des Stickstoffanteiles kann der Methangehalt noch weiter erhöht werden. Für die meisten technischen Einsatzgebiete des gereinigten Biogases (Methangases) ist dies jedoch nicht erforderlich.

Da ausgehend von dem bereits in den Reinigungsstufen K1 bis K3 vorgereinigten Biogas in einer nachfolgenden Aminwäsche nur noch geringe Anteile an Verunreinigungen zu entfernen sind, kann eine Aminwäsche (mit Waschmittelregenerierung) vergleichsweise mit erheblich geringerem Energieaufwand durchgeführt werden, als dies ansonsten bei der Reinigung von Biogas als Rohgas erforderlich ist.

Die erforderliche Wärmeenergie für die Reinigung der aminhaltigen Waschlösung verringert sich somit von 250 kW auf 143 kW. Der spezifische Wärmebedarf bezogen auf die Biogasmenge kann somit von 0,5 auf 0,268 kWh/Nm³ reduziert werden. Ein weiterer Vorteil ist der geringe Methanverlust (0,03%) gegenüber der konventionellen Aminwäsche (0,1 %). Von den eingesetzten 143 kW für die Aminwäsche können durch Wärmerückgewinnung ca. 85% an Wärmeenergie für eine weitere Verwendung wieder zur Verfügung gestellt werden. Diese kann zur Beheizung des Fermenters mit einer Temperatur von 58 °C genutzt werden. Durch Erhöhung der Füllkörperschüttung oder Packungshöhe in der Waschkolonne K1 kann die Beladung des Waschmittels von 800 mg/l auf 1.200 g/l gesteigert werden. Damit kann die CO₂-Abscheidung von 46,4 % auf 83,5 % gesteigert werden. Weiter kann durch Anlagen eines Vakuums in der Strippkolonne K3 von 0,3 anstatt 0,5 bar die Waschmittelbeladung an CO₂ von 300 auf unter 200 mg/l reduziert werden. Dies führt ebenfalls zu einer höheren CO2-Abscheidung. Wird über Leitung 07 in die Vakuumstrippkolonne K3 noch zusätzlich eine geringe Menge von Strippluft mit 1 bis 2 Nm³/h eingeleitet, dann kann des CO₂-Gehalt im Waschwasser bis auf unter 50 mg/ reduziert werden.

## Patentansprüche

1. Verfahren zur Reinigung von methanhaltigem Rohgas, insbesondere Biogas, zur Gewinnung von Methan, wobei im Roh- oder Biogas Kohlendioxid, Schwefelverbin-dungen, Ammoniak und andere organische wasserlösliche Stoffe enthalten sind, die in einem mehrstufigen Reinigungsprozess abgetrennt werden, wobei der Reinigungsprozess in mindestens drei unmittelbar nacheinander folgenden Reinigungsstufen und unter Verwendung von im Kreislauf geführtem zusatzfreien Frischwasser durchgeführt wird, und dabei
a) das zu reinigende Roh- oder Biogas als erste Reinigungsstufe unter Normaldruck oder bei einem Überdruck von bis zu 6 bar eine Waschkolonne (K1) mit Füllkörperschüttung im Gegenstrom zum zugeführten Frischwasser durchströmt und im Frischwasser Kohlendioxid, Schwefelwasserstoff, Ammoniak, und andere im Roh- oder Biogas enthaltene organische wasserlösliche Stoffe gebunden werden, und am Kopf der Waschkolonne (K1) Methangas mit einem Methangehalt von mindestens 60 % abgezogen wird,
b) in der zweiten Reinigungsstufe aus dem aus der Waschstufe (K1) abgeführten verunreinigten Waschwasser in einer ersten Strippkolonne (K2) mit Füllkörperschüttung oder Packung unter Zuführung von Strippluft; Strippluft und Sauerstoff; oder Strippluft und Kohlendioxid; in einer Menge von 0.1 bis 20 %, bezogen auf die Menge an zugeführtem zu reinigenden Roh- oder Biogas, unter Normaldruck, im Gegenstromprinzip, bei Temperaturen von bis zu 80 °C, im Waschwasser gelöstes Methan nahezu vollständig d.h. zu mindestens 90% aus diesem abgetrennt wird und dabei ein sauerstoffhaltiges Strippgas in Brenngasqualität entsteht, und
c) in der dritten Reinigungsstufe aus dem aus der ersten Strippkolonne (K2) abgeführten verunreinigten Waschwasser in einer zweiten Strippkolonne (K3) mit Füllkörperschüttung oder Packung unter Vakuum, im Gleich- oder Gegenstromprinzip zu Strippluft, im Waschwasser gelöstes Kohlendioxid mindestens bis auf einen Restgehalt von unter 300 mg/l abgetrennt, das gereinigte Waschwasser der Waschstufe (K1) zugeführt und das Abgas abgelassen wird.

2. Verfahren nach Anspruch 1, **dadurch gekennzeichnet, dass** das im Kreislauf gefahrene Frischwasser eine Temperatur von bis zu 65 °C aufweist.

3. Verfahren nach einem der Ansprüche 1 oder 2, **dadurch gekennzeichnet, dass** das in der ersten Reinigungsstufe aus der ersten Strippkolonne (K2) abgezogene Strippgas (Abgas) entweder in den Fermenter einer Biogasenlage zurückgeführt oder dem aus der ersten Waschstufe abgetrennten Methangasstrom zugeführt oder als Brenngas genutzt wird.

4. Verfahren nach einem der Ansprüche 1 bis 3, **dadurch gekennzeichnet, dass** in der zweiten Reinigungsstufe die erste Strippkolonne (K2) zur Abtrennung von Methan aus dem verunreinigten Waschwasser zweistufig ausgeführt ist, wobei in der ersten Stufe Sauerstoff und in der zweiten Stufe Strippluft, oder in umgekehrter Reihenfolge, zugeführt, und zwei unterschiedliche Brenngase mit unterschiedlichen Sauerstoffgehalten erhalten werden.

5. Verfahren nach einem der Ansprüche 1 bis 4, **dadurch gekennzeichnet, dass** das in der Waschkolonne (K1) abgezogene Methangas entweder einzeln oder zusammen mit dem aus der ersten Strippkolonne (K2) abgezogenen Strippgas einer weiteren Aufbereitung zur Erhöhung des Methangehaltes zugeführt wird.

6. Verfahren nach einem der Ansprüche 1 bis 5, **dadurch gekennzeichnet, dass** nach einer bestimmten Betriebsdauer, bei einer Anreicherung des Schwefelgehaltes im aus Waschstufe (K1) abgezogenen verunreinigten Waschwasser auf über 50 ppm, das im Kreislauf gefahrene Waschwasser teilweise oder vollständig durch Frischwasser ersetzt wird.

7. Verfahren nach einem der Ansprüche 1 bis 6, **dadurch gekennzeichnet, dass** eine Teilmenge des am Sumpf der zweiten Strippkolonne (K3) abgezogenen Waschwassers ausgekreist, dieser ein Schwefelwasserstoff bindendes Reaktionsmittel zugesetzt wird, und nach dem Ausfällen des Eisen(II)-disulfids das Waschwasser wieder in den Kreislauf zurückgeführt wird.

8. Verfahren nach einem der Ansprüche 1 bis 7, **dadurch gekennzeichnet, dass** mittels der Parameter Waschwassermenge/h und Waschwassertemperatur in der Waschkolonne (K1) die Trennleistung für im Waschwasser gelöstes Kohlendioxid einstellbar ist, wobei eine höhere Waschwassermenge und eine tiefere Waschwassertemperatur zu einer höheren Trennleistung führen.

9. Verfahren nach einem der Ansprüche 1 bis 8, **dadurch gekennzeichnet, dass** das aus der Waschkolonne (K1) abgezogene gereinigte Roh- oder Biogas zur Erhöhung der Methankonzentration und der Speicherkapazität des Biogases im Fermenter direkt in den Fermenter der Biogasanlage eingeleitet wird.

10. Verfahren nach einem der Ansprüche 1 bis 9, **dadurch gekennzeichnet, dass** zur Einstellung des Vakuums in der dritten Reinigungsstufe der Vakuumpumpe (V2) der zweiten Strippkolonne (K3) Falschluft zugeführt wird.

11. Anlage zur Durchführung des Verfahrens nach einem der Ansprüche 1 bis 10, bestehend aus einer als Gaswäscher ausgebildeten Waschkolonne (K1) zur Abtrennung von im Roh- oder Biogas enthaltenen Bestandteile, wie Kohlendioxid, Schwefelverbindungen, Ammoniak und anderer wasserlöslicher Stoffe, mittels Waschwasser, einer ersten Strippkolonne (K2) zur Entfernung von im verunreinigten Waschwasser gelöstem Methan und einer zweiten Strippkolonne (K3), die als Vakuumkolonne ausgelegt ist, zur Entfernung von Kohlendioxid aus dem am Sumpf der ersten Strippkolonne anfallenden verunreinigten Waschwasser, wobei die Waschkolonne und die beiden Strippkolonnen in Reihe geschalten sind, und die Waschkolonne (K1) eine Füllkörperschüttung oder Packung mit einer Oberfläche von 300 bis 900 m²/m³ und einer Schütthöhe von 2 bis 16 m, die erste Strippkolonne (K2) eine Füllkörperschüttung oder Packung mit einer Oberfläche von 350 bis 900 m²/m³ und einer Schütthöhe von 1 bis 4 m und die zweite Strippkolonne (K3) eine Füllkörperschüttung oder Packung mit einer Oberfläche von 100 bis 300 m²/m³ und einer Schütthöhe von 1 bis 10 m enthalten, und der Sumpf der zweiten Strippkolonne (K2) über eine Waschwasser führende Leitung (04) mit dem Kopf der Waschkolonne (K1) verbunden ist, wobei in die Kreislaufleitung (04) eine Pumpe (P1) eingebunden ist.

12. Anlage nach Anspruch 11, **dadurch gekennzeichnet, dass** die Waschkolonne (K1) und die beiden Strippkolonnen (K2, K3) bei gleichem Kolonnendurchmesser unterschiedliche Füllkörperschütthöhen aufweisen, wobei die Verhältnisse der Schütthöhen Waschstufe (K1):erste Strippkolonne (K2):zweite Strippkolonne (K3) 3:1:2 bis 3:0,5:1 betragen.

13. Anlage nach einem der Ansprüche 11 oder 12, **dadurch gekennzeichnet, dass** das Verhältnis der Füllkörperoberflächen erste Strippkolonne(K2):zweite Strippkolonne (K3) 1 :0,2 bis 1:0,B, vorzugsweise 1:0,5, beträgt.

14. Anlage nach einem der Ansprüche 11 bis 13, **dadurch gekennzeichnet, dass** die erste Strippkolonne (K2) in zwei Kolonnenabschnitte (K2A, K2B) unterteilt ist, wobei jeder Kolonnenabschnitt (K2A, K2B) mit einer Füllkörperschüttung oder Packung ausgerüstet ist und der obere Kolonnenabschnitt (K2A) mit einer Sauerstoff zuführenden Leitung (09b) und der untere Kolonnenabschnitt (K2B) mit einer Luft zuführenden Leitung (09a) verbunden ist.

15. Anlage nach einem der Ansprüche 11 bis 14, **dadurch gekennzeichnet, dass** die Trennböden der Waschkolonne (K1) und der Strippkolonnen (K2, K3) hinsichtlich Gasbeladung technisch dicht und hinsichtlich Flüssigkeitsbeladung voll durchlässig ausgeführt sind.

## Claims

1. Method of purifying raw gas, especially biogas, for obtaining methane wherein the components contained in the raw gas or biogas such as carbon dioxide, sulfur compounds, ammonia and other organic water-soluble substances, are removed in a multi-stage purification process, wherein the purification process is carried out in at least three purifying steps taking place in immediate succession to each other and using additive-free fresh water circulated in the circuit and
a) as a first purifying step the raw gas or biogas to be purified (raw gas) drawn off from a plant flows through a scrubbing column (K1) with packed bed under standard pressure or under an overpressure of up to 6 bar in counterflow to the fresh water fed in and the carbon dioxide, hydrogen sulfide, ammonia and other organic water-soluble substances contained in the raw gas or biogas are bound in the fresh water, and methane gas with a methane content of at least 60% is drawn off at the head of the scrubbing column (K1),
b) in the second purifying step the methane dissolved in the contaminated scrubbing solution drawn off from the scrubbing stage K1 is almost completely, i.e. at least 90%, removed under standard pressure on the counterflow principle at temperatures of up to 80°C in a first stripping column (K2) with packed bed or packing with stripping air or stripping air and oxygen or stripping air and carbon dioxide fed in, either singly or in combination, at an amount of 0.1 % to 20% based on the amount of gas fed in, with an oxygenic stripping gas of fuel gas quality being produced in the process, and
c) in the third purifying step the carbon dioxide dissolved in the contaminated scrubbing solution drawn off from the first stripping column (K2) is removed to a residual content of under 300 mg/l in a second stripping column (K3) with packed body or packing under a vacuum in parallel or counterflow to the stripping air, the purified scrubbing solution is fed to the scrubbing stage (K1) and the exhaust gas is drawn off.

2. Method according to claim 1 **characterized in that** the fresh water circulated in the circuit is at a temperature of up to 65°C.

3. Method according to one of the claims 1 or 2 **characterized in that** the stripping gas (exhaust gas) drawn off in the first purifying step from the first stripping column (K2) is either returned to the digester of a biogas plant or fed to the methane gas stream removed in the first scrubbing stage or used as a fuel gas.

4. Method according to one of the claims 1 to 3 **characterized in that** in the second purifying step the first stripping column (K2) for removing methane from the contaminated scrubbing solution is constructed as a two-stage column, with oxygen fed in the first stage and stripping air fed in the second stage, or vice versa, and two different fuel gases with different oxygen contents are produced.

5. Method according to one of the claims 1 to 4 **characterized in that** the methane gas drawn off in the scrubbing column (K1) is fed to a further processing stage to increase the methane content either individually or together with the stripping gas drawn off from the first stripping column (K2).

6. Method according to one of the claims 1 to 5 **characterized in that** the scrubbing solution circulating in the circuit is partly or completely replaced by fresh water after a specified period of operation if the sulfur content in the contaminated scrubbing solution drawn off from scrubbing stage (K1) has risen to a level above 50 ppm.

7. Method according to one of the claims 1 to 6 **characterized in that** a partial amount of scrubbing solution drawn off at the base of the second stripping column (K3) is removed from the circuit, a reactant that binds hydrogen sulfide is added to said solution and the scrubbing solution is returned to the circuit after precipitation of the iron (II) disulfide.

8. Method according to one of the claims 1 to 7 **characterized in that** the capacity to remove the carbon dioxide dissolved in the scrubbing solution can be set by means of the parameters of the amount of scrubbing solution/h and the scrubbing solution temperature in the scrubbing column (K1), with a greater amount of scrubbing solution and a lower scrubbing solution temperature leading to a greater capacity to remove the carbon dioxide.

9. Method according to one of the claims 1 to 8 **characterized in that** the purified raw gas or biogas drawn off from the scrubbing column (K1) to increase the methane concentration and the storage capacity of the biogas in the digester is fed directly to the digester of the biogas plant.

10. Method according to one of the claims 1 to 9 **characterized in that** entrained air is fed to the vacuum pump (V2) in the second stripping column (K3) to create the vacuum in the third purifying step.

11. Plant for carrying out the method according to one of the claims 1 to 10 comprising a scrubbing column (K1) constructed as a gas scrubber for removing by means of scrubbing solution components contained in the raw gas or biogas such as carbon dioxide, sulfur compounds, ammonia and other water-soluble substances a first stripping column (K2) for removing methane dissolved in the contaminated scrubbing solution and a second stripping column (K3) constructed as a vacuum column for removing carbon dioxide from the contaminated scrubbing solution accruing at the base of the first stripping column, wherein the scrubbing column and the two stripping columns are connected in series and the scrubbing column (K1) has a packed bed or packing with a surface area of 300 to 900 m²/m³ and a bed height of 2 to 16 m, the first stripping column (K2) has a packed bed or packing with a surface area of 350 to 900 m²/m³ and a bed height of 1 to 4 m and the second stripping column (K3) has a packed bed or packing with a surface area of 100 to 300 m²/m³ and a bed height of 1 to 10 m, and the base of the second stripping column (K2) is connected to the head of the scrubbing column (K1) by a line (04) carrying a scrubbing solution, with a pump (P1) integrated into the circulation line (04).

12. Plant according to claim 11 **characterized in that** the scrubbing column (K1) and the two stripping columns (K2, K3) have the same column diameter and different packed bed heights, with the ratios of the bed heights in purifying step (K1):first stripping column (K2):second stripping column (K3) being 3:1:2 to 3:0.5:1.

13. Plant according to one of the claims 11 or 12 **characterized in that** the ratio of the surface areas of the packed beds in the first stripping column (K2): second stripping column (K3) is 1:0.2 to 1:0.8, preferably 1:0.5.

14. Plant according to one of the claims 11 to 13 **characterized in that** the first stripping column (K2) is divided into two column sections (K2A, K2B) with each column section (K2A, K2B) fitted with a packed bed or packing and the upper column section (K2A) is connected to a line (09b) feeding in oxygen and the lower column section (K2B) is connected to a line (09a) feeding in air.

15. Plant according to one of the claims 11 to 14 **characterized in that** the separating plates of the scrubbing column (K1) and the stripping columns (K2, K3) are constructed so as to be technically leakproof as regards gas loading and completely permeable as regards fluid loading.

## Revendications

1. Procédé pour l'épuration de gaz bruts contenant du méthane, notamment du biogaz, pour la récupération du méthane, du dioxyde de carbone, des composés de souffre, de l'ammoniaque et d'autres substances organiques solubles dans l'eau, séparés au cours d'un processus d'épuration en plusieurs étapes, étant contenus dans le gaz brut ou le biogaz, le processus d'épuration étant réalisé en au moins trois étapes d'épuration se succédant directement les unes aux autres et en utilisant de l'eau fraîche sans additif, amenée dans le circuit, procédé au cours duquel
a) en tant que première étape d'épuration, le gaz brut ou le biogaz à épurer traverse, sous pression normale ou sous une surpression de 6 bar ou allant jusqu'à 6 bar, une colonne de lavage (K1) à garnissage à contre-courant de l'eau fraîche amenée, et le dioxyde de carbone, l'hydrogène sulfuré, l'ammoniaque et les autres substances organiques solubles dans l'eau, contenues dans le gaz brut ou le biogaz, étant liés dans l'eau fraîche, et le méthane d'une teneur en méthane d'au moins 60% étant éliminé à la tête de la colonne de lavage (K1),
b) au cours de la seconde étape d'épuration, le méthane dissous dans l'eau de lavage est séparé de celle-ci presque entièrement, à savoir au moins à 90%, à partir de l'eau de lavage polluée éliminée de l'étape de lavage (K1) dans une première colonne de stripping (K2) à garnissage ou cartouche avec amenée d'air de stripping ou d'air de stripping plus de l'oxygène ou d'air de stripping plus du dioxyde de carbone, dans une quantité comprise entre 0,1 et 10% par rapport à la quantité de gaz brut ou de biogaz amené à épurer, sous pression normale et à contre-courant, à des températures de 80°C ou allant jusqu'à 80°C, ceci générant un gaz de stripping contenant de l'oxygène présentant la qualité d'un gaz combustible, et
c) au cours de la troisième étape d'épuration, le dioxyde de carbone dissous dans l'eau de lavage est séparé au moins jusqu'à une teneur résiduaire inférieure à 300 mg/l de l'eau de lavage polluée éliminée de la première colonne de stripping (K2) dans une seconde colonne de stripping (K3) à garnissage ou cartouche, sous vide d'air et à courant continu ou contre-courant de l'air de stripping, l'eau de lavage épurée est amenée dans l'étape de lavage (K1) et le gaz résiduaire est rejeté.

2. Procédé selon la revendication 1, **caractérisé en ce que** l'eau fraîche mise en circuit présente une température de 65°C ou allant jusqu'à 65°C.

3. Procédé selon l'une des revendications 1 ou 2, **caractérisé en ce que** le gaz de stripping (gaz résiduaire) éliminé de la première colonne de stripping (K2) au cours de la première étape d'épuration est soit ramené dans le fermenteur d'une installation de production de biogaz soit amené dans le courant de méthane séparé de la première étape de lavage soit utilisé comme gaz combustible.

4. Procédé selon l'une des revendications 1 à 3, **caractérisé en ce qu'**au cours de la seconde étape d'épuration, la première colonne de stripping (K2) pour la séparation du méthane de l'eau de lavage polluée est réalisée en deux niveaux, de l'oxygène étant amené au premier niveau et de l'air de stripping étant amené au second niveau ou inversement, et deux gaz combustibles différents à teneur en oxygène différente sont obtenus.

5. Procédé selon l'une quelconque des revendications 1 à 4, **caractérisé en ce que** le méthane éliminé de la colonne de lavage (K1) est soumis, soit seul soit avec le gaz de stripping éliminé de la première colonne de stripping (K2), à un traitement supplémentaire pour l'augmentation de la teneur en méthane.

6. Procédé selon l'une des revendications 1 à 5, **caractérisé en ce qu'**au terme d'une certaine durée d'exploitation, une fois que l'augmentation de la teneur en souffre de l'eau de lavage polluée éliminée de l'étape de lavage (K1) est supérieure à 50 ppm, l'eau de lavage mise en circuit est remplacée partiellement ou en totalité par de l'eau fraîche.

7. Procédé selon l'une quelconque des revendications 1 à 6, **caractérisé en ce qu'**une quantité partielle de l'eau de lavage éliminée dans le bas de la seconde colonne de stripping (K3) est éliminée du circuit, un agent réactif liant l'hydrogène sulfuré est rajouté à celle-ci, et l'eau de lavage est ramenée dans le circuit au terme de la précipitation du disulfure de fer (II).

8. Procédé selon l'une quelconque des revendications 1 à 7, **caractérisé en ce que** le taux de séparation pour le dioxyde de carbone dissous dans l'eau de lavage peut être réglé au moyen des paramètres de quantité d'eau de lavage/h et de température de l'eau de lavage dans la colonne de lavage (K1), unie quantité d'eau de lavage plus élevée et une température de l'eau de lavage plus basse entraînant un taux de séparation plus élevé.

9. Procédé selon l'une quelconque des revendications 1 à 8, **caractérisé en ce que** le gaz brut ou le biogaz épuré et éliminé de la colonne de lavage (K1) est introduit directement dans le fermenteur de l'installation de production de biogaz afin d'augmenter la concentration en méthane et la capacité de stockage du biogaz dans le fermenteur.

10. Procédé selon l'une quelconque des revendications 1 à 9, **caractérisé en ce que**, pour régler le vide d'air au cours de la troisième étape d'épuration, de l'air parasite est amené dans la pompe à vide (V2) de la deuxième colonne de stripping (K3).

11. Installation pour la mise en oeuvre du procédé selon l'une quelconque des revendications 1 à 10, composée d'une colonne de lavage (K1) réalisée sous forme de laveur de gaz pour la séparation au moyen d'eau de lavage de composants contenus dans le biogaz, comme le dioxyde de carbone, les composés de souffre, l'ammoniaque et d'autres substances solubles dans l'eau, d'une première colonne de stripping (K2) pour l'élimination du méthane dissous dans l'eau de lavage polluée et d'une seconde colonne de stripping (K3) réalisée sous forme de colonne sous vide pour l'élimination du dioxyde de carbone de l'eau de lavage polluée contenue dans le bas de la première colonne de stripping, la colonne de lavage et les deux colonnes de stripping étant montées en série, et la colonne de lavage (K1) comprenant un garnissage ou une cartouche d'une surface comprise entre 300 et 900 m²/m³ et une hauteur de garnissage comprise entre 2 et 16 m, la première colonne de stripping (K2) comprenant un garnissage ou une cartouche d'une surface comprise entre 350 et 900 m²/m³ et une hauteur de garnissage comprise entre 1 et 4 m et la seconde colonne de stripping (K3) comprenant un garnissage ou une cartouche d'une surface comprise entre 100 et 300 m²/m³ et une hauteur de garnissage comprise entre 1 et 10 m, et le bas de la seconde colonne de stripping (K2) étant relié à la tête de la colonne de lavage (K1) via une conduite (04) d'amenée de l'eau de lavage, une pompe (P1) étant intégrée dans la conduite du circuit (04).

12. Installation selon la revendication 11, **caractérisée en ce que** la colonne de lavage (K1) et les deux colonnes de stripping (K2, K3) présentent, pour un diamètre de colonne identique, des hauteurs de garnissage différentes, les rapports des hauteurs de garnissage étape de lavage (K1):première colonne de stripping (K2):seconde colonne de stripping (K3) étant compris entre 3:1:2 et 3:0,5:1.

13. Installation selon l'une quelconque des revendications 11 ou 12, **caractérisée en ce que** le rapport des surfaces des corps de remplissage première colonne de stripping (K2):seconde colonne de stripping (K3) est compris entre 1:0,2 et 1:0,8, de préférence 1:0,5.

14. Installation selon l'une quelconque des revendications 11 à 13, **caractérisée en ce que** la première colonne de stripping (K2) est divisée en deux sections de colonne (K2A, K2B), chaque section de colonne (K2A, K2B) étant équipée d'un garnissage ou d'une cartouche et la section de colonne supérieure (K2A) étant reliée à une conduite (09b) d'amenée d'oxygène et la section de colonne inférieure (K2B) étant reliée à une conduite (09a) d'amenée d'air.

15. Installation selon l'une quelconque des revendications 11 à 14, **caractérisée en ce que** les plateaux de séparation de la colonne de lavage (K1) et des colonnes de stripping (K2, K3) sont réalisés de manière à être techniquement étanches à la charge de gaz et entièrement perméables à la charge de liquide.
